# EUROPEAN PATENT SPECIFICATION

(11) **EP 3 240 599 B1**
(45) Date of publication and mention of the grant of the patent: **09.06.2021**
(21) Application number: 15875953.0
(22) Date of filing: 14.12.2015
(51) Int. Cl.: A61M 16/18

(54) **SYSTEM FOR CONTROLLING PATIENT RECOVERY**
SYSTEM ZUR KONTROLLE DER GENESUNG EINES PATIENTEN
SYSTÈME DE RÉGULATION DE LA RÉCUPÉRATION D'UN PATIENT

(30) Priority: 30.12.2014 GB 201423359
(43) Date of publication of application: 08.11.2017
(73) Proprietor: General Electric Company, Schenectady, NY 12345 (US)
(72) Inventor: HEINONEN, Erkki Paavo, 00510 Helsinki (FI); HAGGBLOM, Tom Jakob, 00510 Helsinki (FI)
(74) Representative: Fennell, Gareth Charles
(86) International application number: PCT/US2015/065581
(87) International publication number: WO 2016/109177

(56) References cited:
- EP-A1- 2 371 410
- EP-A2- 0 720 858
- EP-A2- 0 894 506
- EP-A2- 0 911 052
- US-A- 5 320 093
- US-A1- 2005 051 167
- US-A1- 2005 247 316
- US-A1- 2009 050 148
- US-A1- 2009 050 148
- US-A1- 2012 272 957

## Description

### Technical Field

The present disclosure relates to method and a system for controlling patient recovery from anesthesia with of an inhalation anesthesia agent.

### Background

To allow surgical operation of a patient, a patient is anesthetized to keep the patient free of pain and unconscious as well as to prevent movement of the patient during surgery. The main purpose of general anesthesia is to obtain controlled unconsciousness, pain relief, muscle relaxation and a reduction of the response of the autonomic nervous system.

During general anesthesia a patient is unable to utilize his respiratory muscles for breathing. For this reason, the patient is ventilated with a ventilation device which provides lung gas exchange. The purpose of the ventilation is to supply sufficient oxygen to the patient's lungs. The ventilation also allows exhalation to expel carbon dioxide, which is the waste product of metabolism in humans.

In order to anesthetize a patient, anesthesia agents can be introduced in the body intravenously, for instance, by using a catheter. Alternatively, inhalational induction is possible when using volatile anesthetics. To allow the use of these volatile anesthetics, an anesthesia delivery system comprises in addition to a ventilator a breathing gas mixer and a vaporizer which are used to obtain a required mix of oxygen, inhalation anesthesia agent and balance gas for the ventilation. The most common inhalation anesthesia agents are sevoflurane, desflurane and isoflurane. The balance gas is typically nitrogen (N₂). Alternatively, nitrous oxide (N₂O) can be used. The obtained fresh breathing gas mixture is supplied to a breathing circuit to which the ventilator is connected to forward the mixture to the lungs of a patient. The anesthesia agent in the breathing gas mixture is ventilated into the lungs for diffusion to patient tissues and mediation of the anesthetic effect.

Normally, the patient anesthesia gas concentrations vary from 1% to 10%. From the lungs the anesthesia agent gas dissolves through the alveolar membrane into the blood circulation. The blood circulation carries the agent to the central nervous system. The anesthesia effects start when the concentration of anesthesia reaches a determined level at the central nervous system of the patient.

The inhalation agents are soluble also for other body tissues such as fat. The anesthesia agent diffusion in the body continues until all tissues have reached a required level of agent concentration. The time needed to arrive at this point and the amount of agent needed to reach the required level of concentration, depend on the solubility of the agent used and on the size of the patient.

Patient recovery, this means the wake up of the patient and the recovery of his ability to maintain a sufficient ventilation spontaneously, occurs at the end of an anesthesia process. During the patient recovery there are conflicting objectives to achieve the patient recovery. At first the patient must be cleared from the anesthesia agent to a level that enables the patient to use his muscles. This also means that the concentration of anesthesia agent in all body compartments should decrease to a level which enables muscular activity of the patient.

The time required to remove the anesthesia agent from the patient's body depends on the solubility of the anesthesia agent and effectiveness of the ventilation. This means the time needed to remove the anesthesia agent depends in a first instance on the quantity of anesthesia agent that is dissolved in the body tissues of the patient and in a second instance on the swiftness with which the agent is cleared out using anesthesia agent-free ventilation.

The anesthesia agent-free ventilation is achieved with flushing the ventilation gas circuit with a gas that does not contain the anesthesia agent, as a result whereof the anesthesia agent concentration in the ventilation circuit of a patient will gradually decrease. The agent-free gas is forwarded to the patient lungs will dilute the alveolar gas concentration. A following expiration will expel the agent from the patient in the diluted gas. Diluted alveolar concentration allow diffusion of the agent from the blood to the alveolar gas and onwards the removal of the agent via ventilation. Gradually, the reduced concentration of agent in the blood will help the removal of the agent from all body tissues.

It should be noted that during the agent free ventilation, the ventilation will also have as an effect the clearance of carbon dioxide from the patient lungs. A complicating factor is the fact that the carbon dioxide concentration is the principal activator of the respiratory center of the central nervous system of a human being. For this reason, the carbon dioxide concentration rise in the lungs is central to allowing recovery of a patient. The increase of the carbon dioxide concentration normally starts with apneic ventilation. This means that a clinician stops the ventilation and moves towards manual ventilation control using a manual ventilation bag at a time chosen by the clinician. The reduced ventilation will mean providing less oxygen to the patient and therefore the patient oxygenation may be compromised during this procedure, especially since the body oxygen reservoirs are very small compared to the amount of carbon dioxide. In order to produce carbon dioxide, oxygen is needed for the metabolism of the patient. In other words: oxygen delivery is a prerequisite for carbon accumulation.

Normally, to compensate for the reduced level of oxygen, prior to moving to manual ventilation, the patient lungs are filled with high concentration oxygen which will allow both the lungs and the blood to store oxygen. However, it is possible that the storage of oxygen in the lungs runs empty which results in a declining oxygen supply to the muscles that are needed to start spontaneous breathing of the patient. To reveal the possible oxygen decline, normally the body oxygen saturation is measured during the recovery phase to monitor the need to refill the lungs with oxygen.

The filling of the lungs with a determined quantity of oxygen will have a further adverse effect. The oxygen is dissolved from the lung alveoli into the blood circulation, which depletes the gaseous volume of the alveoli. The alveoli collapse and the blood flow in communication with the closed alveoli does not get oxygenated any more, which compromises the circulatory oxygen delivery. It is possible to recruit the collapsed alveoli by means of high pressure from the ventilator. However, after patient disconnection from the ventilation this option is no longer available. In these circumstances the recruitment of the alveoli will take place only gradually with the development of the patient respiratory muscle strength to generate pleural negative pressure. At the same time the patient will begin to breathe mainly ambient air with supply of oxygen bleed. This may result in a decline of oxygen supply with as an effect the reduction of patient oxygenation and in a worst case hypoxia after disconnection from the ventilation.
US 2012/272957 relates to a system for controlling the delivery of medical gases including a digital signal processor that receives at least one ventilation parameter value change, calculates a fresh oxygen flow rate, total fresh gas flow rate into the breathing circuit, and a reference oxygen flow rate representative of a predetermined oxygen concentration delivered to a patient.
US 2009/050148 relates to an inhalation anesthesia delivery system and method, whereby the system comprises a fresh gas feeding arrangement connected to a breathing circuit, a monitor device, a control device and an interface unit.

In view of the above observations, there appears to be need for an improved method for controlling the recovery of a patient after an inhalation anesthesia procedure to help the patient to recover his ability to maintain sufficient ventilation spontaneously in a controlled and safe manner.

### Summary of the Invention

The present invention is defined in the appended claims. In one aspect, the present disclosure is directed to a a system for controlling patient recovery from anesthesia with an inhalation anesthesia agent, wherein the patient during the recovery is ventilated with a ventilator, the system comprising:
- a breathing device for ventilating a patient, the breathing device comprising tubing to connect the breathing device to a patient's breathing circuit,
- a ventilator connected to the breathing device to provide a pressurized breathing gas,
- a ventilator controller connected to the ventilator to control the gas volume and breath frequency provided with the ventilator,
- a gas mixer and vaporizer for providing patient breathing gas, the gas mixer and vaporizer being connected to the ventilator, the ventilator controller being adapted to detect patient spontaneous breaths and to reduce the ventilator support to match the measured CO₂ concentration in the end-expiration with a set target level,
- a patient gas controller connected to the gas mixer and vaporizer for controlling the composition of the fresh patient breathing gas provided with the gas mixer and vaporizer,
- a sensor connected to the patient gas controller, for providing at least monitored expiratory O₂ concentration for the patient, the patient gas controller being adapted to compare the expiratory O₂ concentration for the patient with a target concentration for said expiratory O₂ concentration and to provide instructions for the gas mixer to control the composition of the patient breathing gas to thereby allow the expiratory O₂ concentration to match said target O₂ concentration.

At least one of the above embodiments provides one or more solutions to the problems and disadvantages with the background art. Other technical advantages of the present disclosure will be readily apparent to one skilled in the art from the following description and claims. Various embodiments of the present application obtain only a subset of the advantages set forth. No one advantage is critical to the embodiments. Any claimed embodiment may be technically combined with any other claimed embodiment(s).

### Brief Description of the Drawings

The accompanying drawings illustrate presently exemplary embodiments of the disclosure and serve to explain, by way of example, the principles of the disclosure.
FIG. 1 is a schematical representation of an anesthesia delivery system according to the present disclosure, and
FIG. 2 shows a flow chart of an example of the method according to the present disclosure.

### Detailed Description

In the present disclosure, the wording "anesthesia inhalation agent" is used to indicate an active substance which can be administered by means of inhalation and which is specifically adapted to anesthetize a patient during surgery. The wording "volatile anesthetic" and the wording "anesthesia inhalation agent" refer to the same type of actives used in inhalation anesthesia procedures.

In the present disclosure, the wording "end-expiration" and "expiration" are used to refer to the gas expired by the patient.

In the present disclosure, the wording "breathing device" is used for a device for ventilating a patient, such as a breathing circuit.

The present disclosure relates to controlling the ventilation and oxygenation of a patient at the end of an anesthesia procedure. This means controlling automatically the ventilation and oxygenation during recovery and reducing the ventilation from a first level, sufficient for gas exchange needed during the period of anesthesia towards disconnection of the patient from the ventilation system, once the patient is able meet the gas exchange demand using spontaneous breathing.

The time of recovery is a time of risk. Normally, patients are observed on a one-to-one basis by an anesthetist or recovery nurse until they have regained airway control and cardiovascular stability and are able to communicate.

The patient is disconnected from a ventilation system and extubated once the patient is conscious, able to maintain a clear airway and exhibits protective airway reflexes. Moreover, the respiration and oxygenation should be at a satisfactory level. In practice measurements are taken to ensure that the patient's cardiovascular system is stable with no unexplained cardiac irregularity or persistent bleeding, with a pulse and blood pressure at an acceptable level.

One objective of the solution according to the present disclosure is to provide an automated system and procedure to allow safe recovery of a patient, wherein the procedure is both safe and operator-independent. In order to allow such a procedure, an anesthesia delivery system is used with a sensor adapted to measure at least the patient end-expiration O₂ concentration. The sensor could be adapted to also measure other end-expiration components such as CO₂ and anesthesia agent concentrations. Moreover, a gas mixer is used for controlling the fresh breathing gas flow rate and flow concentration to the breathing circuit during the recovery to supply oxygen to meet the patient's oxygenation demand. The delivery system further has a ventilator for ventilating patient lungs to transport the breathing gas oxygen to the lungs and to remove carbon dioxide. The ventilation also provides samples of the gas composition in patient's lungs for measurement of the end-expiration concentrations.

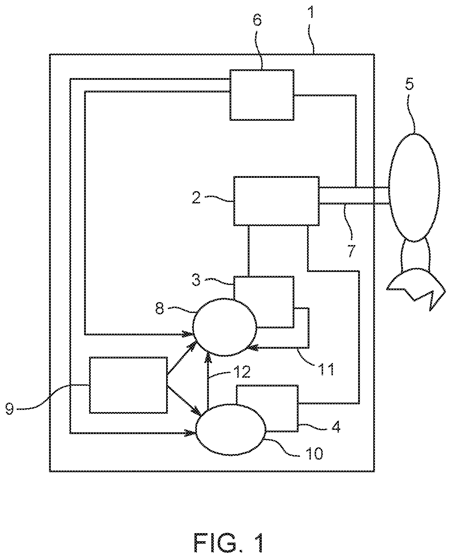

FIG. 1 is a schematical representation of an anesthesia delivery system 1 according to an embodiment of the present disclosure. The anesthesia delivery system 1 comprises a breathing device or breathing circuit 2, a patient limb tubing 7, and a carbon dioxide absorber. Moreover, the system comprises a ventilator 3 to provide ventilation of the airways and lungs of the patient 5 and a gas mixer/vaporizer 4 providing fresh breathing gas to the breathing circuit. A patient monitor 6 is connected to the patient limb tubing 7 connecting the breathing device 2 with the patient 5. The patient monitor 6 comprises a sensor configured to monitor at least the O₂ concentration in a gas flow and is adapted to receive information relating to the gas concentration measurements relating to the expiration of the patient 5. The connection between the patient monitor 6 and the patient limb tubing 7 may comprise a gas sampling line for obtaining a breathing gas sample from the patient limb tubing 7 to allow analysis in a gas analyzer connected to the patient monitor 6. Alternatively, the connection may be electrical if the gas analyzer is directly connected to the patient limb tubing 7.

The breathing circuit 2 will allow gas to be expired from the lungs. Normally, the breathing circuit 2 re-circulates the expired gas towards the ventilator 3 in order to allow the expired gas to be re-used for subsequent ventilation. This is done with the objective to save the relative expensive and environmentally hostile anesthesia components. Before this step of re-circulation of the expired gas, the CO₂ in the gas flow is removed by means of a CO₂ absorber. The remaining gas flow is mixed with a quantity of fresh breathing gas provided by means of the gas mixer/vaporizer 4.

The patient monitor 6 could also be adapted to receive other signals relating to specific physiological parameters of the patent 5, such as the blood hemoglobin values and values relating the oxygen saturation (SpO2) using applicable sensors fitted to the patient 5.

The ventilator 3 may be of any automatically controlled type, such as an electronically controlled type which is known for the use in anesthesia delivery systems. The system 1 further comprises a ventilator controller 8 which is operably connected to the ventilator 3. The ventilator controller 8 is connected to the patient monitor 6, the receive information relating the expiratory gas concentrations. The ventilator controller 8 is further connected to a user interface 9. This user interface 9 is adapted to allow an operator to enter target information, such as a target level for the O₂ and CO₂ in the gas expired by the patient 5. The user interface 9 is further adapted to provide instructions, such as a preferred transition rate of any of the gas components in the patient's expiration. The user interface 9 could also be used to trigger to the start of the automatic patient recovery process.

Based on the input received from both the user interface 9 and the patient controller 6, the ventilation controller 8 is able to generate instructions for the ventilator 3. These instructions may relate to the breath volume, the breath inspiration pressure, the breath frequency and the end expiratory pressure level. The ventilator controller 8 also receives information relating a patient's spontaneous breathing action. This information can be provided via connection 11. In addition, or instead of the connection 11 similar information could be send to the ventilator controller 8 using the patient monitor 6.

If the ventilator controller 8 receives information relating to spontaneous breathing of the patient 5, the ventilation controller 8 can initiate immediate breath support by providing an elevated inspiration pressure. In case the spontaneous breathing action is repeated, the patient 5 takes over the breath frequency and the ventilator controller 8 is able to adjust the support level provided by the ventilator 3 to the frequency imposed by the patient 5. In case the spontaneous breathing would stop, the ventilator controller 8 is adapted to take control of the breathing, including the breathing frequency that is imposed to the patient 5. The ventilator controller 8 continues to receive further signals from the patient gas controller 10 via the connection 12. Through this connection the ventilation controller 8 receives information relating the fresh breathing gas delivery to the patient 5. This information can be used to review whether the ventilation is sufficient from a point of view of patient oxygenation. The maximum fresh breathing gas O₂ concentration in combination with the flow rate and the measured patient end expiratory O₂ concentration provide information about a possible need to increase the ventilation to meet the patient oxygenation demand. In case the measured patient end-expiratory O₂ concentration is below the set target level despite of maximal fresh breathing gas oxygen supply, this will provide an indication that ventilation should be increased.

This ventilation control action includes adjusting the ventilator generated support pressure for patient initiated spontaneous breaths. This control further includes generation of additional breaths if the patient initiated spontaneous breaths are missing for a period of time. For example, 20 seconds is typically used as interval to regard the presence of apnea. This would also provide a sample of patient expiration gas for every 20 seconds to ensure proper patient oxygenation.

The ventilator generated support pressure is controlled also to provide breath volume enabling adequate sample of the patient alveolar gas for valid monitoring of patient expired O2, agent and CO2 concentrations.

Once the patient recovered, the spontaneous actions repeat regularly to control the breathing frequency and the spontaneous breathing actions are sufficiently strong. When the measured end-tidal CO₂ remains under the given recovery CO₂ target the ventilator support can be reduced and finally stopped.

The gas mixer & vaporizer controller 10 receives the gas concentration from the patient monitor 6 and the target levels for O₂ and CO₂ for the recovery process from the user interface 9. The O₂ target may be unchanged from the anesthesia phase target but the CO₂ target is likely increased to allow CO₂ accumulation to stimulate spontaneous breathing. The controller 10 compares the targets with the respective measured values and instructs the gas mixer 4 for O₂ concentration needed to match the target and the ventilator 3 to match both O₂ and CO₂ targets.

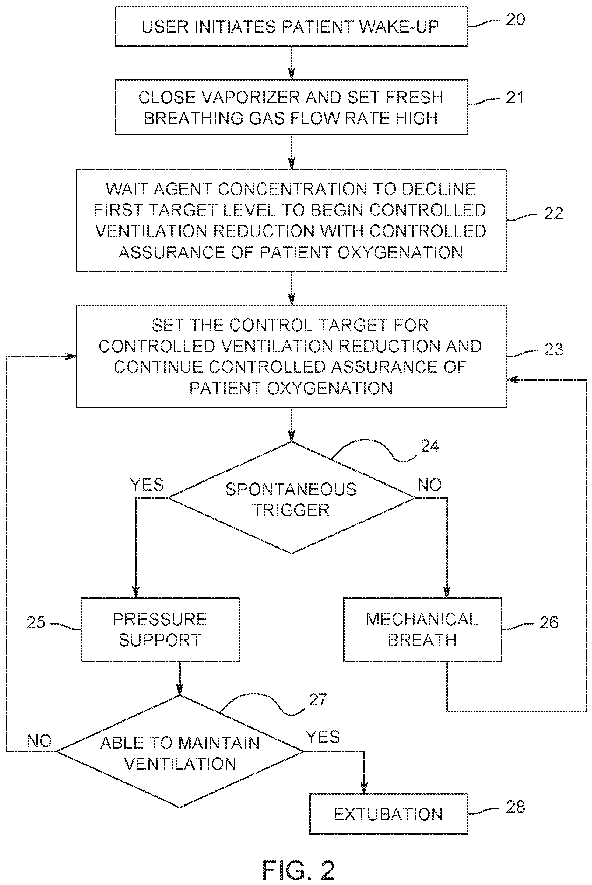

Fig. 2 shows the different steps for automatic patient recovery according to an embodiment of the invention. In a first step 20, the recovery process is initiated. In a further step 21 the concentration of the inhalation anesthesia agent in the ventilation gas is decreased from a first level, adapted for anesthesia purposes, to a second level which is adapted to initiate the patient recovery. This decrease in inhalation anesthesia gas concentration can be obtained by at least partially closing the vaporizer and increasing the fresh breathing gas flow rate. The effect of these measures is that the breathing circuit and alveolar gas will be flushed to remove the anesthesia agent. The O₂ concentration of the flushing flow is controlled to allow the measured O₂ concentration in the end-expiration gas to match with set target value. The ventilation is controlled to match the measured CO₂ value with the given target considering also the oxygenation comparison and oxygen delivery state.

At the same time possible N₂O delivery will be stopped and the balance gas will comprise mainly N₂. The gas mixture will be controlled for the fresh breathing gas O₂ concentration to match the measured end-expiratory O₂ concentration with the target level. The process initiation at step 20 is forwarded to ventilation controller 8 and mixer controller 10 together with related target input data.

The phase of initiating the patient recovery continues until the agent concentration has decreased to a predetermined level that enables the patient to use his muscles. Once the patient is able to breathe spontaneously, the ventilation can be reduced to allow the accumulation of body CO₂, while the O₂ supply is still continued. At the same time, the continued ventilation provides information of the patient gas status in form of new end-expiratory gas concentration measurements. This step is indicated with reference 22 in figure 2. The ventilation controller 8 will begin to monitor the patient inhalation agent concentration to check whether the first target level for agent concentration in the end-expiration is reached. This concentration may be related to the MAC or "minimum alveolar concentration" of the patient which is an agent specific value that describes the depth of patient anesthesia. The level of the MAC is typically: MAC=1, with 50% of the patients not responding to surgical stimulus and being e.g. 0.5*MAC or 0.7 MAC. That can also be defined as MAC-awake, which is 0.3-0.5*MAC. Adult MAC concentrations may vary between different studies, but typical values at sea level pressure are 2% for sevoflurane, 6% for desflurane and 1.2% for isoflurane.

The reduction of the ventilation of the patient may be obtained by either a reduction of breath volume or a reduction in the respiration rate. However, the breath volume must remain significantly larger than the ventilation dead space to allow valid measurements of the end-expiration gas concentrations. Since the breath rate determines how often the patient controller 6 receives information about these gas concentrations, the breath rate must also be maintained above minimum level. Throughout this procedure the patient gas controller 10 has priority to adjust the fresh gas mixture to match the measured O₂ concentration with the given target. However, oxygen delivery requires also ventilation, thus in case the fresh gas controller cannot maintain the O₂ target level, the ventilation may need to be increased.

During this process the ventilator controller 8 regulates the artificial breathing, but also detects and supports patient spontaneous breaths. Initially, the spontaneous breaths are missing and the ventilator controller 8, controls both the breath rate and the breath volume. When the spontaneous breaths start to appear, the spontaneous breaths take over the breath rate control and ventilator controller 8 is used to adjust the breath volume.

As indicated in step 23 of the method, according to figure 2 control targets are set for the ventilation reduction while, the breathing of the patient 5 is controlled to assure the patient oxygenation. The presence of spontaneous breaths is checked in step 24. If such spontaneous breaths do not appear, the ventilator 3 continues to provide ventilation as indicated in step 26. If the spontaneous breaths do appear the ventilator 3 is used to provide pressure support as indicated in step 25. After the step 25, it is monitored whether the patient 5 is able to maintain the spontaneous ventilation in step 27. If the patient 5 is not able to maintain spontaneous ventilation, the method returns to step 23.

Once the first target agent concentration measured in the end-expiration indicates that the patient is ready for spontaneous breathing, the aim is to elevate body CO₂ concentration by setting new target concentrations in order to stimulate spontaneous breathing at respiratory center of the patient's body. Partial pressure required for this is around 6 kPa that corresponds to 6.5% measured end-expiratory dry gas concentration. This set level may be acquired gradually from the target set during anesthesia along with declining agent concentration, or as a single change to the recovery target value.

Gradually, while the spontaneous breathing increases, the ventilator controller 8 reduces the ventilator support to match the measured CO₂ concentration in the end-expiration with the set target level. Finally, the patient 5 can maintain ventilation without ventilator support. An indication for the fact that the patient 5 is able to breath without ventilation support, is given by a measured CO₂ concentration which is kept below a target level, without ventilator support. If the breathing support pressure has arrived at a minimum of, for example, 4 cmH₂O to overcome endotracheal tube flow resistance, regular spontaneous breath rate and the measured end-expiratory CO₂ concentration is kept below the given target, the patient 5 is ready to be disconnected from the ventilation and the tubing can be removed from the patient's body as indicated in step 28 of figure 2.

The method and the system according to the present disclosure help to reduce the risk for post-operative oxygenation problems due to atelectasis. This reduction of risk is realized by the fact that the patient lungs no longer need filled with high concentration oxygen prior to interruption of ventilation.

### LIST OF ELEMENTS

1 = anesthesia delivery system
2 = breathing device
3 = ventilator
4 = gas mixer & vaporizer
5 = patient
6 = patient monitor
7 = tubing
8 = ventilator controller
9 = user interface
10 = patient gas controller
11 = connection
12 = connection
20 = first step of method
21 = second step of method
22 = third step of method
23 = fourth step of method
24 = fifth step of method
25 = sixth step of method
26 = seventh step of method
27 = eighth step of method
28 = ninth step of method

## Claims

1. System for controlling patient recovery from anesthesia with an inhalation anesthesia agent, wherein the patient during the recovery is ventilated with a ventilator, the system comprising:
- a breathing device (2) for ventilating a patient, the breathing device comprising tubing to connect the breathing device to a patient's breathing circuit,
- a ventilator (3) connected to the breathing device to provide a pressurized breathing gas,
- a ventilator (8) controller connected to the ventilator (3) to control the gas volume and breath frequency provided with the ventilator, the ventilator controller being adapted to detect patient spontaneous breaths and to reduce the ventilator support to match the measured CO₂ concentration in the end-expiration with a set target level,
- a gas mixer and vaporizer for providing patient breathing gas, the gas mixer and vaporizer being connected to the ventilator,
- a patient gas controller (10) connected to the gas mixer and vaporizer for controlling the composition of the fresh patient breathing gas provided with the gas mixer and vaporizer,
- a sensor connected to the patient gas controller (10), for providing at least monitored expiratory O₂ concentration for the patient, the patient gas controller being adapted to compare the expiratory O₂ concentration for the patient with a target concentration for said expiratory O₂ concentration and to provide instructions for the gas mixer to control the composition of the patient breathing gas to thereby allow the expiratory O₂ concentration to match said target O₂ concentration,
wherein the sensor is adapted to determine the expiratory CO₂ concentration and is connected to the ventilator controller (8) to allow the ventilator controller (8) to control the ventilation pressure and frequency provided by means of the ventilator (3).

2. System according to claim 1 , wherein the sensor is adapted to determine the occurrence of spontaneous breathing.

## Patentansprüche

1. System zur Kontrolle der Erholung eines Patienten von der Anästhesie mit einem Inhalationsanästhetikum, wobei der Patient während der Erholung mit einem Beatmungsgerät beatmet wird, wobei das System umfasst:
- eine Atemvorrichtung (2) zum Beatmen eines Patienten, wobei die Atemvorrichtung Schlauchmaterial zum Verbinden der Atemvorrichtung mit einem Atemkreislauf eines Patienten umfasst,
- ein Beatmungsgerät (3), das mit der Atemvorrichtung verbunden ist, um ein druckbeaufschlagtes Atemgas bereitzustellen,
- eine Beatmungsgerätsteuerung (8), die mit dem Beatmungsgerät (3) verbunden ist, um das Gasvolumen und die Atemfrequenz zu steuern, die mit dem Beatmungsgerät bereitgestellt werden, wobei die Beatmungsgerätsteuerung eingerichtet ist, um Spontanatemzüge des Patienten zu detektieren und die Beatmungsgerätunterstützung zu reduzieren, um die gemessene CO₂-Konzentration in der Endexspiration mit einem voreingestellten Zielniveau abzugleichen,
- einen Gasmischer und Verdampfer zum Bereitstellen von Patientenatemgas, wobei der Gasmischer und Verdampfer mit dem Beatmungsgerät verbunden sind,
- eine Patientengassteuerung (10), die mit dem Gasmischer und Verdampfer verbunden ist, um die Zusammensetzung des frischen Patientenatemgases zu steuern, das mit dem Gasmischer und Verdampfer bereitgestellt wird,
- einen Sensor, der mit der Patientengassteuerung (10) verbunden ist, um mindestens überwachte exspiratorische O₂-Konzentration für den Patienten bereitzustellen, wobei die Patientengassteuerung eingerichtet ist, um die exspiratorische O₂-Konzentration für den Patienten mit einer Zielkonzentration für die exspiratorische O₂-Konzentration zu vergleichen und Anweisungen an den Gasmischer bereitzustellen, um die Zusammensetzung des Patientenatemgases zu steuern, um dadurch zu ermöglichen, dass die exspiratorische O₂-Konzentration der Ziel-O₂-Konzentration entspricht,
wobei der Sensor eingerichtet ist, um die exspiratorische CO₂-Konzentration zu ermitteln, und mit der Beatmungsgerätsteuerung (8) verbunden ist, um zu ermöglichen, dass die Beatmungsgerätsteuerung (8) den Beatmungsdruck und die Beatmungsfrequenz steuert, die mittels des Beatmungsgeräts (3) bereitgestellt werden.

2. System nach Anspruch 1, wobei der Sensor eingerichtet ist, um das Auftreten von Spontanatemzügen zu ermitteln.

## Revendications

1. Système de régulation de la récupération d'un patient après une anesthésie avec un agent anesthésiant par inhalation, le patient pendant la récupération étant ventilé avec un insufflateur, le système comprenant :
- un dispositif respiratoire (2) pour la ventilation d'un patient, le dispositif respiratoire comprenant un tube pour relier le dispositif respiratoire à un circuit respiratoire du patient,
- un insufflateur (3) relié au dispositif respiratoire pour fournir un gaz respiratoire sous pression,
- un dispositif de régulation d'insufflateur (8) relié à l'insufflateur (3) pour réguler le volume de gaz et la fréquence de respiration fournis par l'insufflateur, le dispositif de régulation d'insufflateur étant adapté pour détecter les respirations spontanées du patient et pour réduire l'assistance de l'insufflateur afin de faire correspondre la concentration de CO₂ mesurée dans l'expiration finale avec un niveau cible défini,
- un mélangeur et vaporisateur de gaz pour fournir un gaz respiratoire au patient, le mélangeur et vaporisateur de gaz étant relié à l'insufflateur,
- un dispositif de régulation de gaz de patient (10) relié au mélangeur et vaporisateur de gaz pour réguler la composition du gaz respiratoire frais de patient fourni par le mélangeur et vaporisateur de gaz,
- un capteur relié au dispositif de régulation de gaz de patient (10), pour fournir au moins une concentration d'O₂ expiré surveillée pour le patient, le dispositif de régulation de gaz de patient étant adapté pour comparer la concentration d'O₂ expiré pour le patient avec une concentration cible pour ladite concentration d'O₂ expiré et pour fournir des instructions au mélangeur de gaz pour réguler la composition du gaz de respiration du patient pour permettre ainsi à la concentration d'O₂ expiré de correspondre à ladite concentration d'O₂ cible,
le capteur étant adapté pour déterminer la concentration de CO₂ expiré, et étant relié au dispositif de régulation d'insufflateur (8) pour permettre au dispositif de régulation d'insufflateur (8) de réguler la pression et la fréquence de ventilation fournies au moyen de l'insufflateur (3).

2. Système selon la revendication 1,
le capteur étant adapté pour déterminer la survenue de la respiration spontanée.
